Europäisches Patentamt

European Patent Office

Office européen des brevets

(11) Veröffentlichungsnummer: **0 300 346 B1**

(19)

(12) **EUROPÄISCHE PATENTSCHRIFT**

(45) Veröffentlichungstag der Patentschrift: **26.08.92**

(21) Anmeldenummer: **88111204.9**

(22) Anmeldetag: **13.07.88**

(51) Int. Cl.⁵: **C07C 29/136**, C07C 31/125

(54) **Verfahren zur Hydrierung von Fettsäuremethlyestern im Druckbereich von 20 bis 100 bar.**

(30) Priorität: **22.07.87 DE 3724254**

(43) Veröffentlichungstag der Anmeldung:
**25.01.89 Patentblatt 89/04**

(45) Bekanntmachung des Hinweises auf die
Patenterteilung:
**26.08.92 Patentblatt 92/35**

(84) Benannte Vertragsstaaten:
**AT BE CH DE ES FR GB GR IT LI LU NL SE**

(56) Entgegenhaltungen:
**EP-A- 0 254 189**
**FR-A- 1 276 722**
**US-A- 2 109 844**

(73) Patentinhaber: **Henkel Kommanditgesellschaft
auf Aktien
Postfach 1100 Henkelstrasse 67
W-4000 Düsseldorf-Holthausen(DE)**

(72) Erfinder: **Fleckenstein, Theo, Dr.
Pfitznerstrasse 9
W-4010 Hilden(DE)**
Erfinder: **Pohl, Joachim, Dr.
Leinenweberweg 23
W-4000 Düsseldorf(DE)**
Erfinder: **Carduck, Franz-Josef, Dr.
Landstrasse 18
W-5657 Haan(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur katalytischen Hydrierung von Fettsäuremethylestern unter Verwendung stückiger und/oder gekörnter Kupferchromit enthaltender Katalysatoren im Druckbereich von 20 bis 100 bar.

Fettalkohole, d.h. überwiegend lineare, monofunktionelle Alkohole mit Kettenlägen von 8 und mehr C-Atomen, sowie ihre Herstellung sind in der Literatur ausführlich beschrieben, beispielsweise in Ullmanns Encyklopädie der technischen Chemie, 4. Auflage, Band 11, Seiten 427 bis 445. Ein bevorzugtes Ausgangsmaterial zu ihrer Gewinnung sind die in Fetten und/oder Ölen natürlichen Ursprungs vorkommenden Fettsäuren bzw. Fettsäuregemische, die durch katalytische Hydrierung in Fettalkohole entsprechender Kettenlänge umgewandelt werden können. Durch den Einsatz der zu reduzierenden Fettsäuren in Form ihrer Methylester werden insbesondere die Katalysatoren vor einem aggressiven Angriff durch die freie Carboxylgruppe geschützt, so daß in großtechnischen Verfahren für hinreichend lange Zeiträume mit befriedigenden Raumzeitausbeuten gearbeitet werden kann. Demgemäß wird die überwiegende Menge nativer Fettalkohole heute aus Fettsäuremethylestern nach dem Gasphasenhydrierverfahren hergestellt, bei dem die destillierten Methylester über fest angeordnete, kupferhaltige Mischoxid-Katalysatoren, wie beispielsweise Kupferchromit-Spinellkatalysatoren, in dampfförmigem Zustand zusammen mit einem großen Überschuß an Wasserstoff bei Temperaturen von oberhalb 200°C und Drucken von etwa 250 bis 300 bar geleitet werden.

Die auf feuchtem Wege durch gemeinsame Fällung hergestellten Kupfer-Mischoxid-Katalysatoren werden als stückige Kontakte oder Strangpreßlinge benutzt und vor Gebrauch meist in der Anlage reduziert.

In der einschlägigen Patentliteratur werden daher häufig Fettsäureester, insbesondere Fettsäuremethylester und freie Fettsäuren gleichzeitig als Einsatzmaterialien für die Hydrierungsreaktion zu gesättigten und/oder ungesättigten Fettalkoholen verwendet. Verwiesen sei beispielsweise auf die DE-PSen 965 236, 10 05 497, 25 13 377 und 26 13 226. Für die großtechnische praktische Verwertung sind die genannten Vorschläge durchaus unterschiedlich zu bewerten, je nachdem, ob die Fettsäureester oder die freien Fettsäuren als Ausgangsmaterial der Hydrierung verwendet werden.

In der **FR-A 1 276 722** ist ein Verfahren zur Herstellung eines Katalysators beschrieben, der für die Hydrierung von Estern von aliphatischen Carbonsäuren mit $C_1$-$C_6$-Monoalkoholen eingesetzt werden kann. Es wird vorgeschlagen, bekannte Katalysatormassen, die Kupferoxid, Chromoxid und gegebenenfalls Bariumoxid enthalten, unter Zuhilfenahme eines wasserlöslichen Bindemittels in stückige Form zu bringen, danach das Bindemittel vollständig auszulaugen, bevor der Katalysator in die Hydrierung eingesetzt wird. Angaben über die spezifische Oberfläche oder das Porenvolumen sind dieser Druckschrift nicht zu entnehmen; es wird jedoch davor gewarnt, bei der Verformung Graphit einzusetzen. Für die Herstellung von abriebfesten Katalysatoren und die Minimierung von Nebenprodukten in der Hydrierung von Fettsäuremethylestern bietet das Dokument keine Anhaltspunkte.

Die Patentschrift **US 2,109,844** beschreibt die katalytische Direkthydrierung von Fettsäureglycerinestern zu den entsprechenden Alkoholen und enthält ebenfalls weder einen Hinweis auf die Herstellung von abriebfesten Katalysatoren noch oder darauf, wie die Bildung von Nebenprodukten in der Hydrierung von Fettsäuremethylestern minimiert werden kann.

Die DE-OS 34 25 758 beschreibt ein Verfahren zur Herstellung von Alkoholen, insbesondere entweder Furfurylalkohol durch Hydrierung von Furfural, oder Fettalkohol durch Hydrierung von Fettsäuren mit einer entsprechenden Anzahl an C-Atomen oder deren Estern, bei einem Druck im Bereich von 20 bis 100 bar, bei einer Temperatur im Bereich von 150 bis 300 °C und in Anwesenheit eines Katalysators, der eine Kupfer-Chrom-Mischung einerseits und Kupfer auf einem Träger andererseits umfaßt.

Bei den genannten Bedingungen wird in der Praxis auch eine Reduktion der erzeugten Fettalkohole zu den entsprechenden Kohlenwasserstoffen beobachtet, die die Wirtschaftlichkeit des Verfahrens aufgrund einer Ausbeuteminderung in Frage stellt. Ein weiterer Nachteil ist darin zu sehen, daß die üblicherweise verwendeten Katalysatoren nur ungenügende mechanische Festigkeit aufweisen, so daß nicht nur die Abtrennung des festen Katalysators von den Reaktionsprodukten ausgesprochen schwierig ist, sondern auch durch Abschwemmen von Katalysatormaterial Verluste an aktivem Katalysator in Kauf genommen werden müssen.

Es wurde nun überraschend gefunden, daß es mit bestimmten, Kupferchromit als Hauptbestandteil enthaltenden Katalysatoren bei vergleichsweise moderaten Reaktionsbedingungen, insbesondere bei relativ niedrigem Reaktionsdruck, mit hoher Aktivität und unter langer Standzeit der Katalysatoren möglich ist, die Hydrierung von Fettsäuremethylestern zu Fettalkoholen so zu steuern, daß keine bzw. nur sehr wenig Kohlenwasserstoffe als Nebenprodukte gebildet werden.

Aufgabe der vorliegenden Erfindung war es, ein Verfahren zur katalytischen Hydrierung von Fettsäuremethylestern unter Verwendung stückiger und/oder gekörnter, Kupferchromit enthaltender Katalysatoren zur Verfügung zu stellen, mit dem Fettsäuremethylester bei relativ niedrigen Drucken in hoher Ausbeute zu Fettalkoholen umgesetzt werden können. Der für die Umsetzung verwendete heterogene Übergangsmetall-Katalysator sollte mit hoher Aktivität und Selektivität zu den gewünschten Produkten führen, ohne daß Folgereaktionen, wie Kohlenwasserstoffbildung, in nennenswertem Maß zu einer Verminderung der Produktausbeute beitragen. Zusammen mit einer Einstellung schonender Reaktionsbedingungen sollte damit die Wirtschaftlichkeit des Verfahrens im Vergleich zum Stand der Technik verbessert werden.

Die möglichst geringen Anteile an Kohlenwasserstoffen sind erwünscht, da bei einer destillativen Aufarbeitung der Hydrierprodukte Überschneidungen der Siedebereiche der Kohlenwasserstoffprodukte mit denen der kurzkettigen Fettalkohole auftreten.

Die Erfindung betrifft ein Verfahren zur katalytischen Hydrierung von Fettsäuremethylestern bei erhöhten Reaktionstemperaturen unter Verwendung stückiger und/oder gekörnter, Kupferchromit als Hauptbestandteil enthaltender Katalysatoren, das dadurch gekennzeichnet ist, daß man Fettsäuremethylester zusammen mit Wasserstoff bei Drucken von 20 bis 100 bar und Temperaturen von 160 bis 270°C bei Molverhältnissen von Wasserstoff : Fettsäuremethylester-Einsatz von 10 : 1 bis 500 : 1 kontinuierlich über Katalysatoren umsetzt, die 30 bis 40 Gew.-% Kupfer, 23 bis 30 Gew.-% Chrom, 1 bis 10 Gew.-% Mangan 1 bis 10 Gew.% Silicium und 1 bis 7 Gew.-% Barium sowie gegebenenfalls zusätzlich 1 bis 5 Gew.% Zirkon und/oder Cer - Gew.-% jeweils bezogen auf oxidische Katalysatormasse - enthalten und nach dem Calcinieren der die Katalysatormasse bildenden Komponenten mit 1 bis 10 Gew.-% - bezogen aufs oxidischen Katalysator - wenigstens eines Binders und 1 bis 10 Gew.-% Graphit zu stückigen und/oder grobkörnigen Formlingen, die eine spezifische Oberfläche im Bereich von 30 bis 50 m$^2$/g, ein Porenvolumen im Bereich von 0,4 bis 0,6 cm$^3$/g, und bei Stückigen Formlingen einen Durchmesser von 1 bis 6 mm und eine Länge von 1 bis 6 mm, bei gekörnten Formlingen eine Korngröße im Bereich von 0,6 bis 3,0 mm aufweisen, umgewandelt und mit Wasserstoff oder einem Wasserstoff enthaltenden Gasgemisch aktiviert worden sind.

Gemäß dem erfindungsgemäßen Verfahren katalytisch hydrierbare Fettsäuremethylester können nativen oder synthetischen Ursprungs sein. Als Ausgangsstoffe für das erfindungsgemäße Verfahren zur Hydrierung kommen die aus tierischen oder pflanzlichen Quellen entstammenden Fette, Trane oder Öle in Frage, in denen einfach oder mehrfach ungesättigte Fettsäuren mit Glycerin verestert sind, wobei die Fettsäurereste gleiche oder unterschiedliche Sättigungsgrade bzw. Längen ihrer Alkylketten aufweisen können. Die Fettsäuremethylester können durch bekannte Verfahren aus den genannten Fetten, Tranen und Ölen durch Umesterung erhalten werden. Derartige Methylestergemische werden durch Destillationsverfahren in kürzer- und längerkettige Fettsäurereste aufgetrennt und anschließend hydriert.

Gemäß dem erfindungsgemäßen Verfahren wird die katalytische Hydrierung der Fettsäuremethylester in Gegenwart eines Katalysators durchgeführt, der - bezogen auf die oxidische Katalysatormasse - 30 bis 40 Gew.-% Kupfer, 23 bis 30 Gew.-% Chrom, 1 bis 10 Gew.-% Mangan, 1 bis 7 Gew.-% Barium und 1 bis 10 Gew.% Silicium enthält. Die genannten Metalle liegen nach der Herstellung der Katalysatormassen, die auf an sich aus dem Stand der Technik bekannte Art und Weise erfolgt, in Form ihrer Oxide vor. Die Oxidbildung erfolgt, wie aus dem Stand der Technik bekannt, im Verlauf des sogenannten "Calcinierens", d.h. durch thermische Zersetzung von Mineralsalzen der jeweiligen Metalle.

In einer bevorzugten Ausführungsform des erfindungsgemäßen Verfahrens hydriert man kontinuierlich Fettsäuremethylester unter Verwendung eines Katalysators, der vorteilhafterweise 32 bis 38 Gew.-% Kupfer, bezogen auf die oxidische Katalysatormasse, enthält. Ebenso kann es bevorzugt sein, die Menge an Chrom in dem verwendeten Katalysator auf einen Bereich von 26 bis 29 Gew.-%, die Menge an Mangan auf einen Bereich von 1 bis 6 Gew.-%, die Menge an Barium auf einen Bereich von 1,5 bis 3 Gew.-% oder die Menge an Silicium auf einen Bereich von 1,5 bis 3 Gew.-%, jeweils bezogen auf die oxidische Katalysatormasse vor der Aktivierung einzustellen. In einer besonders bevorzugten Ausführungsform wird zur katalytischen Hydrierung von Fettsäuremethylestern ein Katalysator verwendet, der 32 bis 38 Gew.-% Kupfer, 26 bis 29 Gew.-% Chrom, 1 bis 6 Gew.-% Mangan, 1,5 bis 3 Gew.-% Barium, 1,5 bis 3 Gew.% Silicium und 2 bis 3 Gew.% Zirkon und/oder Cer jeweils bezogen auf die oxidische Katalysatormasse vor der Aktivierung enthält. Mit derartigen Katalysatoren lassen sich erhebliche Aktivitätssteigerungen auch bei relativ niedrigen Drucken erzielen. Die Verwendung derartiger, zusätzlich dotierter Katalysatoren in dem Verfahren führt insbesondere bei der Hydrierung im Rieselbett zu einer erheblichen Erhöhung der Aktivität und Selektivität der Katalysatoren. Insbesondere kann die Kohlenwasserstoff-Bildung unterdrückt werden. Aus diesem Grunde ist die Verwendung eines derartigen Katalysators in dem erfindungsgemäßen Verfahren als besonders bevorzugt anzusehen.

Erfindungsgemäße Katalysatoren können 1 bis 10 Gew.-% an Graphit zur verbesserten Verarbeitbarkeit der Granulat- und/oder Extrudatkörper enthalten. Vorzugsweise wird eine Menge von 5 Gew.-% Graphit vor der Granulierung dem calcinierten pulverförmigen Material zugesetzt und mit diesem innig vermischt.

Erfindungsgemäß wurde eine Verbesserung des Verfahrens dadurch erreicht, daß der verwendete, die oben genannten Metalle in Form ihrer Oxide und Graphit enthaltende Katalysator unter Einsatz von 1 bis 10 Gew.-% eines oder mehrerer Binder in Granulat- oder Extrudatform gebracht wird. Bevorzugt wird dabei ein Einsatz von 10 Gew.-% eines oder mehrerer Binder. Als solche kommen für diesen Zweck aus dem Stand der Technik bekannte Verbindungen in Frage, wobei in dem erfindungsgemäß verwendeten Katalysator entweder ein oder auch mehrere Binder verwendet werden können. Besonders hat sich der Einsatz eines oder mehrerer Binder aus Polyvinylacetat und Methylmethacrylat bewährt. Im Gegensatz zu zahlreichen, aus dem Stand der Technik bekannten, schlecht rieselfähigen Katalysatormaterialien konnte für das erfindungsgemäße Verfahren ein Katalysator in Granulat- oder Extrudatform zur Verfügung gestellt werden, dessen aufgelockerte, poröse Struktur zur Erhöhung der Aktivität und Selektivität des Katalysators bei der Hydrierung bei relativ niedrigen Drucken der Fettsäuremethylester insbesondere im Rieselbett in erheblichem Maße beiträgt. Bevorzugt wird als Binder für die Herstellung der Katalysatorgranulate oder Katalysatorextrudate Polyvinylacetat, wobei zur Katalysatorherstellung beispielsweise 40 Gew.-%ige Polyvinylacetat-Suspensionen verwendet werden, die im Handel erhältlich sind. Die calcinierten pulverförmigen Katalysatormaterialien werden nach guter Durchmischung mit derartigen Polyvinylacetat-Suspensionen in kleinen Mengen versetzt und bis zum Beginn des Aufbaus von Agglomeratkörnern gemischt. Im Anschluß daran wird das Agglomerate enthaltende Pulver beispielsweise in einem Lochwalzengranulator zu kleinen Granulaten verdichtet, wobei dieser Prozeß an sich aus dem Stand der Technik bekannt ist. Auf ebenfalls an sich bekannte Weise werden die Granluate getrocknet, wobei sich Restfeuchten von 10 bis 15 % einstellen lassen. Die aus diesem Vorgang resultierenden Granulate werden gesiebt, wobei für das erfindungsgemäße Verfahren Kornfraktionen bestimmter Korngröße ausgesiebt werden. Bei der Anwendung des erfindungsgemäßen Verfahrens zur katalytischen Hydrierung von Fettsäuremethylestern in der Rieselfahrweise werden mit Vorteil Katalysator-Kornfraktionen eingesetzt, deren Korngröße im Bereich von 0,6 bis 3,0 mm liegt.

Die Katalysatoren können auch in Tablettenform, beispielsweise der Größe 4 x 4 mm, gepreßt werden. Zur Härtung dieser Tabletten werden diese 6 h bei einer Temperatur von 200 bis 280°C an der Luft getempert. Die nach der BET-Methode (Z. Anal. Chem. 288 (1968), 187-193) bestimmte spezifische Oberfläche betrug 40 ± 10 m$^2$/g.

Die in dem erfindungsgemäßen Verfahren zur Hydrierung von Fettsäuremethylestern verwendbaren granulierten Katalysatoren weisen eine spezifische Oberfläche im Bereich von 30 bis 50 m$^2$/g auf. Die beschriebene Art der Vorgranulierung führt zu einer speziellen, aufgelockerten Porenstruktur, die sich in einer Erhöhung des Porennutzungsgrades auswirkt.

Im Verlaufe der Untersuchungen zu dem erfindungsgemäßen Verfahren zur Hydrierung von Fettsäuremethylestern hat es sich gezeigt, daß es besonders bevorzugt ist, die Fettsäuremethylester in Gegenwart eines Katalysators mit Wasserstoff umzusetzen, dessen Granulat-, Extrudat- oder Tablettenkörper einen Durchmesser von 1 bis 6 mm und eine Länge von 1 bis 6 mm aufweisen. Derartige Granulat- bzw. Extrudatkörper (Tabletten) zeigen eine ausgezeichnete Aktivität und Selektivität bei der Umsetzung der Fettsäuremethylester mit Wasserstoff zu langkettigen Fettalkoholen und lassen sich zudem ohne Probleme von den Reaktionsprodukten abtrennen. Außerdem sind die mit diesen Katalysatoren erzielbaren Standzeiten deutlich besser als die Standzeiten der aus dem Stand der Technik bekannten Katalysatoren, die zudem den Nachteil aufwiesen, daß sie zum Teil bei der Reaktion zerfielen und dadurch nur unter großen Problemen von den Reaktionsprodukten abtrennbar waren.

Ein weiterer Faktor, der die Aktivität bzw. Selektivität der erfindungsgemäß eingesetzten Katalysatoren stark beeinflußt, ist das Porenvolumen der Katalysator-Formkörper. Es zeigte sich, daß das Porenvolumen der erfindungsgemäß verwendbaren Katalysatoren in einem Optimalbereich liegen muß, um in dem erfindungsgemäßen Verfahren zur Hydrierung von Fettsäuremethylestern optimale Ergebnisse zu erbringen. In einer bevorzugten Ausführungsform des erfindungsgemäßen Verfahrens werden metallhaltige Katalysatoren verwendet, deren Porenvolumen im Bereich von 0,4 bis 0,6 cm$^3$/g liegt. Vorteilhafterweise trägt ein Porenvolumen in diesem Bereich ebenfalls zu einer Erhöhung der Aktivität und Selektivität der Hydrierkatalysatoren bei: Sowohl im Rieselbettreaktor als auch im Sumpfphasenreaktor lassen sich hohe Aktivitäten und Selektivitäten erzielen; gleichzeitig wiesen derartige Katalysatoren in dem erfindungsgemäßen Verfahren eine ausgesprochen hohe Standzeit auf und führten nicht zu Problemen bei der Trennung von Katalysator und Reaktionsprodukten.

Die in dem erfindungsgemäßen Verfahren verwendeten Katalysatoren werden üblicherweise vor ihrem Einsatz in der Hydrierung von Fettsäuremethylestern mit Wasserstoff oder einem Wasserstoff enthaltenden Gasgemisch aktiviert. Aus ökonomischen Gründen wird mit Vorteil zur Aktivierung der Katalysatormassen ein Gasgemisch verwendet, das überwiegend aus einer Stickstoff-/Wasserstoff-Gasmischung besteht. Vorteilhafterweise kann eine derartige Aktivierung - wie aus dem Stand der Technik bekannt - so durchgeführt werden, daß die Katalysatormassen nach der Herstellung im Stickstoffstrom bei erhöhter Temperatur getrocknet werden und dem trocknenden Gas in steigenden Mengen Wasserstoff zur Aktivierung beigemischt wird. Dabei kann der Wasserstoffanteil in dem aktivierenden Gasgemisch im Bereich von 0,1 bis 10 Vol.-% liegen. Die Aktivierung der Katalysatoren kann dabei sowohl in situ als auch in von dem Reaktionsgefäß getrennten Gefäßen durchgeführt werden.

Die Reaktionstemperaturen der Hydrierung von Fettsäuremethylestern gemäß der vorliegenden Erfindung liegen im Bereich von 160 bis 270°C, bevorzugt im Bereich von 180 bis 240°C. Bei der Temperaturführung der Reaktion ist allgemein zu beachten, daß die Hydrierung der Fettsäuremethylester zu Fettalkoholen eine exotherme chemische Reaktion ist. Bei der Temperaturführung muß also darauf geachtet werden, daß nach "Anspringen" der Reduktion der Fettsäuremethylester die entstehende Reaktionswärme in üblicher Weise abzuführen ist.

Das erfindungsgemäße Verfahren zur katalytischen Hydrierung von Fettsäuremethylestern wird bei Reaktionsdrucken im Bereich von 20 bis 100 bar durchgeführt. Der bevorzugte Bereich der Reaktionsdrukke beträgt 20 bis 50 bar. Bei diesen relativ niedrigen Reaktionsdrucken ist eine hohe Aktivität und Selektivität der Katalysatoren sichergestellt, so daß die Raum-/Zeitausbeute für das erfindungsgemäße Verfahren in einem optimalen Bereich liegt.

Das erfindungsgemäße Verfahren zur Hydrierung von Fettsäuremethylestern ist außerdem dadurch gekennzeichnet, daß man ein Molverhälntis von Wasserstoff zu Fettsäuremethylester-Substrat von 10 : 1 bis 500 : 1 einstellt.

Die Vorteile des erfindungsgemäßen Verfahrens sind darin zu sehen, daß durch eine Optimierung sowohl der chemischen Zusammensetzung als auch des physikalischen Aufbaus der verwendeten Katalysatoren auf Kupferchromit-Basis und die verminderten Reaktionsdrucke die Selektivität des Verfahrens, insbesondere hinsichtlich der Unterdrückung der Kohlenwasserstoff-Bildung, verbessert werden konnte.

Die Erfindung wird durch die nachfolgenden Beispiele näher erläutert.

Ausführungsbeispiel

Herstellung eines Katalysators:

Bei Temperaturen von 30 bis 90°C wurden 84,93 g $Ba(NO_3)_2$, 3493 g $Cu(NO_3)_2$ . 2 $H_2O$, 294,43 g $Mn(NO_3)_2$ . 4 $H_2O$ und 62,3 g $SiO_2$ in Form eines 40 Gew.%igen Kieselsäuresols in 9 l entsalztem Wasser unter starkem Rühren gelöst. In einem zweiten Behälter wurden 1639 g $CrO_3$ in 9 l entsalztem Wasser unter den gleichen Bedingungen gelöst und anschließend 3650 g einer 25 %igen Ammoniaklösung hinzugegeben. Danach wurde die Barium, Mangan und Kupfer enthaltende Lösung bei Temperaturen von 30 bis 90°C in die vorgelegte Ammoniumchromatlösung gepumpt, wobei aus der Lösung ein Gemenge von Bariumchromat, Manganhydroxid, Siliciumhydroxid und Kupferchromat ausfiel. Die Fällung war beendet bei Absinken des pH-Wertes unter 7.

Das Präzipitat wurde in einer Rahmenfilterpresse abfiltriert und mit entsalztem Wasser nitratfrei gewaschen. Der Filterkuchen wurde über Nacht bei 90 bis 120°C getrocknet und danach in einer Schneidmühle zu grobem Pulver gemahlen. Das resultierende Chromatpulver wurde im Drehrohrofen bei Temperaturen von 300 bis 500°C thermisch zum Chromit zersetzt ("calciniert"). Das calcinierte, pulverförmige Material hatte folgende chemische Zusammensetzung:
Cu: 38 ± 0,5 %;
Cr: 29 ± 0,5 %;
Mn: 2,5 ± 0,5 %;
Ba: 1,9 ± 0,5 % und
Si: 1 ± 0,3 %.

Einem Liter des Pulvers wurden 5 Gew.-% Graphit zugesetzt und im Lödige-Mischer 15 min durchmischt. Anschließend wurden 10 Gew.-% einer 40 gew.-%igen Polyvinylacetatsuspension zugegeben und kurz bis zum Aufbaubeginn von Agglomeraten durchgemischt. Im Anschluß daran wurde das Pulver in einem Lochwalzengranulator zu kleinen Granulaten verdichtet, auf eine Restfeuchte von 10 bis 15 % getrocknet und zu einer 0,6- bis 2 mm-Kornfraktion gesiebt.

Das Granulat hatte ausgezeichnete Fließeigenschaften und konnte auf einer Rundläufer-Tablettenmaschine zu Tabletten von 3 bis 6 mm Durchmesser und 2 bis 4 mm Höhe verpreßt werden.

Nach der Härtung (6h, 200°C, an der Luft) resultierten Tabletten mit einer spezifischen Oberfläche (bestimmt nach BET) von 40 ± 10 $m^2$/g und einem Porenvolumen von 0,4 bis 0,6 $cm^3$/g.

Beispiele 1 bis 5 und Vergleichsbeispiel 1

In einem 1 l Rieselreaktor, der mit Katalysatortabletten (Durchmesser 4 mm; Höhe 4 mm) gemäß dem Herstellungsbeispiel beschickt war, wurde kontinuierlich ein Gemisch von Fettsäuremethylestern mit Kettenlängen von 12 bis 18 C-Atomen im Gleichstrom mit Wasserstoff umgesetzt.

Die flüssigen und kondensierbaren Reaktionsprodukte wurden durch eine zweistufige Abscheidung in Druckgefäßen aufgefangen.

Die Verfahrensparameter sowie die erzielten Ergebnisse sind der nachfolgenden Tabelle zu entnehmen.

Tabelle

| | Beispiele | | | | | Vgl. 1 |
|---|---|---|---|---|---|---|
| | 1 | 2 | 3 | 4 | 5 | |
| Reaktionsdruck (bar) | 50 | 50 | 30 | 30 | 20 | 10 |
| Reaktionstemperatur (°C) | 200 | 205 | 200 | 250 | 200 | 250 |
| LHSV* ($l \times l^{-1} \times h^{-1}$) | 1,0 | 1,0 | 0,5 | 1,0 | 0,5 | 0,5 |
| $H_2$ : Substrat ($mol \times mol^{-1}$) | 100 | 200 | 100 | 200 | 100 | 100 |
| Produkt-Verseifungszahl | 4,5 | 2,8 | 0,8 | 0,7 | 2,5 | 3,1 |
| Produkt-Zusammensetzung (Gew.-%) | | | | | | |
| Fettalkohole | 84,3 | 84,9 | 85,6 | 83,3 | 84,8 | 79,4 |
| Kohlenwasserstoffe | 0,06 | 0,07 | 0,12 | 2,36 | 0,25 | 5,5 |
| Methanol | 13,7 | 13,8 | 14,0 | 13,9 | 13,8 | 13,8 |

LHSV* (liquid hourly space velocity)

## Patentansprüche

1. Verfahren zur katalytischen Hydrierung von Fettsäuremethylestern bei erhöhter Reaktionstemperatur unter Verwendung stückiger und/oder gekörnter, Kupferchromit als Hauptbestandteil enthaltender Katalysatoren, **dadurch gekennzeichnet,** daß man Fettsäuremethylester zusammen mit Wasserstoff

7

bei Drücken von 20 bis 100 bar und Temperaturen von 160 bis 270°C bei Molverhältnissen von Wasserstoff : Fettsäuremethylester-Einsatz von 10 : 1 bis 500 : 1 kontinuierlich über Katalysatoren umsetzt, die

30 bis 40 Gew.-% Kupfer,
23 bis 30 Gew.-% Chrom,
1 bis 10 Gew.-% Mangan
1 bis 10 Gew.-% Silicium und
1 bis 7 Gew.-% Barium
sowie gegebenenfalls zusätzlich
1 bis 5 Gew.-% Zirkon und/oder Cer
  - jeweils bezogen auf oxidische Katalysatormasse - enthalten
und nach dem Calcinieren der die Katalysatormasse bildenden Komponenten mit 1 bis 10 Gew.-% - bezogen auf oxidischen Katalysator - wenigstens eines Binders und 1 bis 10 Gew.-% - bezogen auf oxidischen Katalysator - Graphit zu stückigen und/oder gekörnten Formlingen, die eine spezifische Oberfläche im Bereich von 30 bis 50 $m^2/g$, ein Porenvolumen im Bereich von 0,4 bis 0,6 $cm^3/g$, und bei stückigen Formlingen einen Durchmesser von 1 bis 6 mm und eine Länge von 1 bis 6 mm, bei gekörnten Formlingen eine Korngröße im Bereich von 0,6 bis 3,0 mm aufweisen, umgewandelt und mit Wasserstoff oder einem Wasserstoff enthaltenden Gasgemisch aktiviert worden sind.

2. Verfahren nach Anspruch 1, **dadurch gekennzeichnet,** daß man Fettsäuremethylester einsetzt.

3. Verfahren nach den Ansprüchen 1 und 2. **dadurch gekennzeichnet,** daß man Fettsäuremethylester unter Verwendung eines 32 bis 38 Gew.-% - bezogen auf die oxidische Katalysatormasse - Kupfer enthaltenden Katalysators einsetzt.

4. Verfahren nach den Ansprüchen 1 und 2. **dadurch gekennzeichnet,** daß man Fettsäuremethylester unter Verwendung eines 26 bis 29 Gew.-% - bezogen auf die oxidische Katalysatormasse - Chrom enthaltenden Katalysators einsetzt.

5. Verfahren nach den Ansprüchen 1 und 2. **dadurch gekennzeichnet,** daß man Fettsäuremethylester unter Verwendung eines 1,5 bis 3 Gew.-% - bezogen auf die oxidische Katalysatormasse - Barium enthaltenden Katalysators einsetzt.

6. Verfahren nach den Ansprüchen 1 und 2. **dadurch gekennzeichnet,** daß man Fettsäuremethylester unter Verwendung eines 1,5 bis 3 Gew.-% - bezogen auf die oxidische Katalysatormasse - Silicium enthaltenden Katalysators einsetzt.

7. Verfahren nach den Ansprüchen 1 und 2, **dadurch gekennzeichnet,** daß man Fettsäuremethylester unter Verwendung eines Katalysators einsetzt, der - bezogen auf die oxidische Katalysatormasse -
32 bis 38 Gew.-% Kupfer,
26 bis 29 Gew.-% Chrom,
1 bis 6 Gew.-% Mangan,
1,5 bis 3 Gew.-% Barium und
1,5 bis 3 Gew.-% Silicium
enthält.

8. Verfahren nach den Ansprüchen 1 bis 7, **dadurch gekennzeichnet,** daß man Fettsäuremethylester unter Verwendung eines Katalysators einsetzt, der - bezogen auf die oxidische Katalysatormasse -
32 bis 38 Gew.-% Kupfer,
26 bis 29 Gew.-% Chrom,
1 bis 6 Gew.-% Mangan,
1,5 bis 3 Gew.-% Barium,
1,5 bis 3 Gew.-% Silicium und
2 bis 3 Gew.-% Zirkon und/oder Cer
enthält.

9. Verfahren nach den Ansprüchen 1 bis 8, **dadurch gekennzeichnet,** daß man als Binder Polyvinylacetat und/oder Methylmethacrylat einsetzt.

**10.** Verfahren nach den Ansprüchen 1 bis 9, **dadurch gekennzeichnet,** daß man den Katalysator mit einem 0,1 bis 10 Vol.-% Wasserstoff enthaltenden $N_2$-/$H_2$-Gasgemisch aktiviert.

**11.** Verfahren nach den Ansprüchen 1 bis 10, **dadurch gekennzeichnet,** daß man die Hydrierung bei 180 bis 240°C durchführt.

**Claims**

**1.** A process for the catalytic hydrogenation of fatty acid methyl esters at elevated reaction temperatures using pelleted and/or granular catalysts containing copper chromite as principal constituent, characterized in that fatty acid methyl esters are continuously reacted with hydrogen under pressures of 20 to 100 bar and at temperatures of 160 to 270°C with molar ratios of hydrogen to fatty acid methyl ester substrate of from 10:1 to 500:1, the reaction being carried out over catalysts which contain
30 to 40% by weight copper,
23 to 30% by weight chromium,
1 to 10% by weight manganese,
1 to 10% by weight silicon and
1 to 7% by weight barium
and, optionally,
1 to 5% by weight zirconium and/or cerium
(% by weight, based in each case on oxidic catalyst mass)
and which, after calcination of the components forming the catalyst mass, have been converted into pellets and/or granules having a specific surface of 30 to 50 $m^2$/g, a pore volume of 0.4 to 0.6 $cm^3$/g and, in the case of pellets, a diameter of 1 to 6 mm and a length of 1 to 6 mm, in the case of granules a grain size of 0.6 to 3.0 mm, with 1 to 10% by weight, based on oxidic catalyst, of at least one binder and 1 to 10% by weight, based on oxidic catalyst, graphite and activated with hydrogen or a hydrogen-containing gas mixture.

**2.** A process as claimed in claim 1, characterized in that fatty acid methyl ester is used.

**3.** A process as claimed in claims 1 and 2, characterized in that fatty acid methyl ester is hydrogenated using a catalyst containing 32 to 38% by weight, based on oxidic catalyst mass, of copper.

**4.** A process as claimed in claims 1 and 2, characterized in that fatty acid methyl ester is hydrogenated using a catalyst containing 26 to 29% by weight, based on oxidic catalyst mass, of chromium.

**5.** A process as claimed in claims 1 and 2, characterized in that fatty acid methyl ester is hydrogenated using a catalyst containing 1.5 to 3% by weight, based on oxidic catalyst mass, of barium.

**6.** A process as claimed in claims 1 and 2, characterized in that fatty acid methyl ester is hydrogenated using a catalyst containing 1.5 to 3% by weight, based on oxidic catalyst mass, of silicon.

**7.** A process as claimed in claims 1 and 2, characterized in that fatty acid methyl ester is hydrogenated using a catalyst containing
32 to 38% by weight copper,
26 to 29% by weight chromium,
1 to 6% by weight manganese,
1.5 to 3% by weight barium and
1.5 to 3% by weight silicon,
based on oxidic catalyst mass.

**8.** A process as claimed in claims 1 to 7, characterized in that fatty acid methyl ester is hydrogenated using a catalyst containing
32 to 38% by weight copper,
26 to 29% by weight chromium,
1 to 6% by weight manganese,

1.5 to 3% by weight barium,
1.5 to 3% by weight silicon and
2 to 3% by weight zirconium and/or cerium,
based on oxidic catalyst mass.

9. A process as claimed in claims 1 to 8, characterized in that polyvinyl acetate and/or methyl methacrylate is/are used as binder.

10. A process as claimed in claims 1 to 9, characterized in that the catalyst is activated with an $N_2$-/$H_2$ gas mixture containing 0.1 to 10% by volume hydrogen.

11. A process as claimed in claims 1 to 10, characterized in that the hydrogenation is carried out at 180 to 240°C.

**Revendications**

1. Procédé pour l'hydrogénation catalytique d'esters méthyliques d'acides gras à haute température de réaction, avec utilisation de catalyseurs en morceaux et/ou en grains contenant de la chromite de cuivre en tant que composant principal, caractérisé en ce que l'on fait réagir un ester méthylique d'acide gras conjointement avec de l'hydrogène sous des pressions de 20 à 100 bars et à des températures de 160 à 270°C, à des rapports molaires du mélange hydrogène : ester méthylique d'acide gras allant de 10 : 1 à 500 : 1, en continu, sur des catalyseurs qui contiennent :
    de 30 à 40 % en poids de cuivre,
    de 23 à 30 % en poids de chrome,
    de 1 à 10 % en poids de manganèse,
    de 1 à 10 % en poids de silicium et
    de 1 à 7 % en poids de baryum
ainsi qu'éventuellement additionnellement
    de 1 à 5 % en poids de zirconium et/ou de cérium,
dans chaque cas par rapport à la masse de catalyseur sous forme d'oxyde,
et après la calcination des composants constituant la masse de catalyseur, qui ont été convertis avec 1 à 10 % en poids, par rapport au catalyseur sous forme d'oxyde, d'au moins un liant et 1 à 10 % en poids, par rapport au catalyseur sous forme d'oxyde, de graphite, en comprimés en morceaux et/ou en grains, présentant une surface spécifique dans la plage de 30 à 50 $m^2$/g, un volume de pores dans la plage de 0,4 à 0,6 $cm^3$/g, et, dans le cas de comprimés en morceaux, un diamètre de 1 à 6 mm et une longueur de 1 à 6 mm, dans le cas de comprimés en grains, une taille de grains dans la plage de 0,6 à 3,0 mm, et qui ont été activés avec de l'hydrogène ou avec un mélange gazeux contenant de l'hydrogène.

2. Procédé selon la revendication 1, caractérisé en ce que l'on met en réaction des esters méthyliques d'acides gras.

3. Procédé selon les revendications 1 et 2, caractérisé en ce que l'on met en réaction des esters méthyliques d'acides gras en utilisant un catalyseur contenant de 32 à 38 % en poids de cuivre, par rapport à la masse de catalyseur sous forme d'oxyde.

4. Procédé selon les revendications 1 et 2, caractérisé en ce que l'on met en réaction des esters méthyliques d'acides gras en utilisant un catalyseur contenant de 26 à 29 % en poids de chrome, par rapport à la masse du catalyseur sous forme d'oxyde.

5. Procédé selon les revendications 1 et 2, caractérisé en ce que l'on met en réaction des esters méthyliques d'acides gras en utilisant un catalyseur contenant de 1,5 à 3 % en poids de baryum , par rapport à la masse de catalyseur sous forme d'oxyde.

6. Procédé selon les revendications 1 et 2, caractérisé en ce que l'on met en réaction des esters méthyliques d'acides gras en utilisant un catalyseur contenant de 1,5 à 3 % en poids de silicium, par rapport à la masse de catalyseur sous forme d'oxyde.

**7.** Procédé selon les revendications 1 et 2, caractérisé en ce que l'on met en réaction des esters méthyliques d'acides gras en utilisant un catalyseur qui contient, par rapport à la masse de catalyseur sous forme d'oxyde :

de 32 à 38 % en poids de cuivre

de 26 à 29 % en poids de chrome,

de 1 à 6 % en poids de manganèse,

de 1,5 à 3 % en poids de baryum et

de 1,5 à 3 % en poids de silicium.

**8.** Procédé selon l'une quelconque des revendications 1 à 7, caractérisé en ce que l'on met en réaction des esters méthyliques d'acides gras en utilisant un catalyseur qui contient, par rapport à la masse de catalyseur sous forme d'oxyde :

de 32 à 38 % en poids de cuivre,

de 26 à 29 % en poids de chrome,

de 1 à 6 % en poids de manganèse,

de 1,5 à 3 % en poids de baryum,

de 1,5 à 3 % en poids de silicium et

de 2 à 3 % en poids de zirconium et/ou de cérium.

**9.** Procédé selon l'une quelconque des revendications 1 à 8, caractérisé en ce que l'on utilise en tant que liant, du poly(acétate de vinyle) et/ou du méthacrylate de méthyle.

**10.** Procédé selon l'une quelconque des revendications 1 à 9, caractérisé en ce que l'on active le catalyseur avec un mélange gazeux $n_2/H_2$ contenant de 0,1 à 10 % en volume d'hydrogène.

**11.** Procédé selon l'une quelconque des revendications 1 à 10, caractérisé en ce que l'on effectue l'hydrogènation à une température de 180 à 240°C.